# EUROPÄISCHE PATENTANMELDUNG

(11) **EP 0 789 017 A1**
(43) Veröffentlichungstag der Anmeldung: **13.08.1997**
(21) Anmeldenummer: 97100728.1
(22) Anmeldetag: 17.01.1997
(51) Int. Cl.: C07D 229/00, C09D 175/04, C08G 18/02, C08G 18/10, C08G 18/79

(54) **Uretdiondiisocyanate**

(30) Priorität: 30.01.1996 DE 19603245
(71) Anmelder: BAYER AG, 51368 Leverkusen (DE)
(72) Erfinder: Laas, Hans-Josef, Dr., 50733 Köln (DE); Halpaap, Reinhard, Dr., 51519 Odenthal (DE); Pedain, Josef, Dr., 51061 Köln (DE); König, Klaus, Dr., 51519 Odenthal (DE)

(57) **Zusammenfassung**

Reine Bis(isocyanatoalkyl)-monouretdione, deren Herstellung sowie deren Verwendung als Ausgangskomponente für Polyurethankunststoffe, insbesondere als Isocyanatkomponente in Polyurethanlacken.

## Beschreibung

Die Erfindung betrifft ein Verfahren zur Herstellung reiner Bis(isocyanatoalkyl)monouretdione, diese Bis(isocyanatoalkyl)monouretdione sowie deren Verwendung als Ausgangskomponente für Polyurethankunststoffe, insbesondere als Isocyanatkomponente in Polyurethanlacken, sowie Lacke enthaltend diese so hergestellten Verbindungen.

Die Herstellung oligomerer aliphatischer Polyisocyanate, die Uretdionstrukturen enthalten, durch katalytische Oligomerisierung eines Teils der Isocyanatgruppen monomerer aliphatischer Diisocyanate, Abbruch der Reaktion bei einem gewünschten Umsatzgrad und Entfernen des überschüssigen nicht umgesetzten Diisocyanates durch Dünnschichtdestillation ist bekannt. Ein umfassender Überblick über die technisch relevanten Dimerisierungsverfahren des Standes der Technik findet sich in J. prakt. Chem. 336 (1994) 185-200.

Aufgrund der Difunktionalität der Ausgangsverbindungen fallen bei allen bekannten Verfahren zur Herstellung Uretdionstrukturen enthaltender Polyisocyanate die Reaktionsprodukte nicht in Form reiner Verbindungen an, sondern immer als Gemische von Oligomeren, die eine Molekulargewichtsverteilung aufweisen. Die Zusammensetzung dieser Gemische, die je nach Art des eingesetzten Dimerisierungskatalysators neben Uretdionstrukturen auch mehr oder weniger große Mengen an Isocyanuratstrukturen enthalten können, ist unmittelbar abhängig vom Modifizierungsgrad der Ausgangsmonomeren, sodaß sich Produkteigenschaften, wie mittleres Molekulargewicht, mittlere Isocyanatfunktionalität und Viskosität, innerhalb gewisser Bereiche gezielt einstellen lassen. Während ein hoher Modifizierungsgrad, gleichbedeutend mit einer Erhöhung des mittleren Molekulargewichtes, beispielsweise eine Viskositätszunahme bewirkt, erhält man bei geringerem Umsatz größere Anteile an niedermolekularen Oligomeren.

Reine, d.h. aus genau zwei Molekülen Ausgangsdiisocyanat aufgebaute Monouretdiondiisocyanate, lassen sich nach den bekannten Verfahren, nicht herstellen. Gerade aber solche "dimeren Diisocyanate", die frei von höhermolekularen Anteilen sind, sind aufgrund der zu erwartenden besonders niedrigen Viskosität z. B. als Vernetzer für lösemittelarme Zweikomponenten-Polyurethanlacke von großem Interesse.

Aufgabe der vorliegenden Erfindung war es daher, Bis(isocyanatoalkyl)-monouretdione in reiner Form zur Verfügung zu stehen und ein Verfahren zu ihrer Herstellung anzugeben.

Die Erfindung liegt die Beobachtung zugrunde, daß sich aliphatische, aus genau zwei Molekülen Diisocyanat aufgebaute Monouretdiondiisocyanate durch Vakuumdestillation in geeigneten handelsüblichen Dünnschichtverdampfern bei Temperaturen oberhalb von 160°C aus an sich bekannten monomerenarmen Oligomerenmischungen isolieren lassen, ohne daß in größerem Maße eine Rückpaltung der Uretdionstrukturen in freie Isocyanatgruppen eintritt. Dies ist überraschend, weil die katalytische Dimerisierung von Isocyanaten bekanntlich thermisch reversibel ist, weshalb Uretdione auch als "intern blockierte" Isocyanatgruppen angesehen werden können. Als Deblockierungs-, d. h. Aufspalttemperatur von Uretdiongruppen wird in der Literatur im allgemeinen eine Temperatur im Bereich von 160°C angegeben (z. B.: P. Müller et al.; Angew. Makromol. Chem. 65 (1977) 23; F. Schmitt, XIX. Fatipec Kongess, Vol. III, Aachen 1988, S. 211ff). Oberhalb dieser Temperatur muß daher in erheblichem Maße mit Uretdionspaltung gerechnet werden. Aus diesem Grund wendet man auch bei der Herstellung uretdiongruppenhaltiger oligomerer Polyisocyanate zur Abtrennung der nicht umgesetzten Ausgangsmonomeren möglichst schonende Destillationsbedingungen an (vgl. z. B. EP-A 178 520 und EP-A 337 116).

Gegenstand der vorliegenden Erfindung ist ein Verfahren zur Herstellung von reinen Bis(isocyanatoalkyl)-monouretdionen der Formel (I) 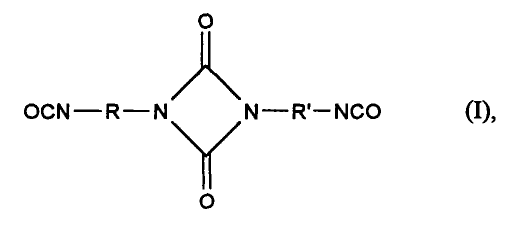 in welcher
- R und R': für gleiche oder verschiedene, lineare oder verzweigte Alkylreste mit 4 bis 9 Kohlenstoffatomen stehen,
durch Oligomerisierung eines Teils der Isocyanatgruppen von Diisocyanaten oder Mischungen von Diisocyanaten der Formel (II)

OCN―R―NCO (II),

in welcher
- R: die bei Formel (I) genannte Bedeutung hat,
in Gegenwart von die Dimerisierung von Isocyanatgruppen beschleunigenden Katalysatoren oder Katalysatorsystemen, Abbruch der Oligomerisierungsreaktion bei einem gewünschten Oligomerisierungsgrad und Entfernung des nicht umgesetzten Diisocyanatüberschusses durch Extraktion oder Dünnschichtdestillation und anschließender Ablagerung der Bis(isocyanatoalkyl)-monouretdione durch Dünnschicht-Destillation bei 160 bis 220°C und einem Druck von 0,01 bis 1.0 mbar.

Ein weiterer Gegenstand sind die so erhaltenen reinen Bis(isocyanatoalkyl)monouretdione der Formel I.

Gegenstand der Erfindung ist auch die Verwendung dieser Bis(isocyanatoalkyl)monouretdione zur Herstellung von Polyurethankunststoffen, insbesondere, gegebenenfalls in mit Blockierungsmitteln für Isocyanatgruppen blockierter Form, als Isocyanatkomponente in Polyurethanlacken.

Ausgangsmaterialien für das erfindungsgemäße Verfahren sind beliebige aliphatische Diisocyanate der Formel (II) oder beliebige Gemische solcher Diisocyanate, wie z.B. 1,4-Diisocyanatobutan, 1,6-Diisocyanatohexan (Hexamethylendiisocyanat; HDI), 1,5-Diisocyanato-2,2-dimethylpentan und 2,2,4- bzw. 2,4,4-Trimethyl-1,6-diisocyanatohexan. Bevorzugt kommen beim erfindungsgemäßen Verfahren Diisocyanate der oben genannten Formel zum Einsatz, in denen der Rest R für einen linearen oder verzweigten Alkylrest mit 4 bis 6 Kohlenstoffatomen steht. Ganz besonders bevorzugt ist die Verwendung von HDI.

Als Katalysatoren für das erfindungsgemäße Verfahren sind grundsätzlich alle bekannten, die Dimerisierung alphatischer Isocyanatgruppen katalysierenden Verbindungen geeignet, beispielsweise tertiäre organische Phosphine (z.B. US-A 46 14 785, Kolonne 4, Zeilen 11 bis 47; DE-A 19 34 763 oder DE-A 39 00 053), peralkylierte Aminophosphine (z. B. DE-A 30 30 513, DE-A 32 27 779 oder DE-A 34 37 635) und 4-Dialkylamino-substituierte Pyridine (DE-A 37 39 549) aber auch Antimonpentafluorid (DE-A 34 20 114) oder Bortrifluorid (DE-A 16 70 720).

Bevorzugt kommen beim erfindungsgemäßen Verfahren als Katalysatoren dreiwertigen Phosphor enthaltende Verbindungen, wie die genannten tertiären organischen Phosphine oder peralkylierten Aminophosphine zum Einsatz. Ganz besonders bevorzugte Katalysatoren sind Tributylphosphin oder Trioctylphosphin.

Die beim erfindungsgemäßen Verfahren eingesetzten Katalysatoren werden im allgemeinen in Mengen von 0,01 bis 5 Gew.-%, vorzugsweise 0,1 bis 2 Gew.-%, bezogen auf die Menge des eingesetzten Ausgangsdiisocyanates, eingesetzt.

Gegebenenfalls können neben den genannten Katalysatoren beim erfindungsgemäßen Verfahren auch geeignete Cokatalysatoren mitverwendet werden. Hierbei handelt es sich beispielsweise um beliebige organische Verbindungen, die mindestens ein an Sauerstoff, Stickstoff oder Schwefel gebundenes Wasserstoffatom und einen pKₛ von mindestens 6 aufweisen, wie sie z. B. in der DE-A 34 37 635, Seite 11, Zeile 8 bis Seite 16, Zeile 6 beschrieben sind.

Als Cokatalysatoren werden niedermolekulare, ein- oder mehrwertige Alkohole, d.h. insbesondere solche des Molekulargewichtsbereiches 32 bis 200 bzw. beliebige Gemische derartiger Alkohole bevorzugt. Geeignet sind beispielsweise Methanol, Ethanol, n-Propanol, Isopropanol, n-Butanol, n-Hexanol, 2-Ethyl-1-hexanol, 1-Methoxy-2-propanol, Ethylenglykol, Propylenglykol, die isomeren Butandiole, Hexandiole, oder Octandiole, Diethylenglykol, Dipropylenglykol, 2-Ethyl-1,3-hexandiol, 2,2,4-Trimethylpentandiol, Glycerin, Trimethylolpropan oder beliebige Gemische derartiger Alkohole.

Diese Cokatalysatoren kommen beim erfindungsgemäßen Verfahren, falls überhaupt, in Mengen von bis zu 5 Gew.-%, vorzugsweise von 0,5 bis 3 Gew.-%, bezogen auf die Menge an eingesetztem Ausgangsdiisocyanat, zum Einsatz.

Die durch Reaktion der beim erfindungsgemäßen Verfahren gegebenfalls mitzuverwendenen Cokatalysatoren mit dem Ausgangsdiisocyanat gebildeten Umsetzungsprodukte stellen die eigentlichen Cokatalysatoren dar. Hieraus folgt, daß prinzipiell anstelle der beispielhalt genannten Cokatalysatoren auch deren separat hergestellte Umsetzungsprodukte mit Ausgangsdiisocyanat, beispielsweise durch Umsetzung der bevorzugt eingesetzten alkoholischen Cokatalysatoren mit Diisocyanat erhaltene Urethane, als Cokatalysatoren geeignet sind.

Zum Abbruch der Oligomerisierungsreaktion geeignete Katalysatorgifte stellen beispielsweise Alkylierungsmittel wie Dimethylsulfat oder p-Toluolsulfonsäuremethylester, Acylierungsmittel wie Benzoylchlorid, Säuren wie Perfluorbutansulfonsäure, Schwefel oder Sulfonylisocyanate dar, wie sie in der US-PS 4 614 785, Kolonne 5, Zeile 27 bis Kolonne 6, Zeile 35 beispielhaft genannt sind. Als Katalysatorgifte ebenfalls geeignet sind silylierte Säuren (EP-A 520 210) sowie Oxidationsmittel wie organische (Hydro)peroxide, Peroxycarbonsäuren oder Sauerstoff (EP-A 481 318).

Die zur Abstoppung der Reaktion benötigte Menge des Katalysatorgiftes richtet sich nach der Menge des verwendeten Katalysators; im allgemeinen wird eine äquimolare Menge des Abstoppers, bezogen auf den zu Beginn eingesetzten Dimerisierungskatalysator, eingesetzt. Berücksichtigt man allerdings eventuell während der Reaktion auftretende Katalysatorverluste, so können zur Reaktionsstoppung auch schon 20 bis 80 Äquivalent-% des Katalysatorgiftes, bezogen auf die ursprünglich eingesetzte Katalysatormenge, ausreichen.

Gegebenenfalls, beispielsweise bei Einsatz der obengenannten substituierten Pyridinkatalysatoren, ist es sogar möglich, völlig auf die Verwendung eines Katalysatorgiftes zu verzichten. Der Katalysator wird dann bei Erreichen des angestrebten Oligomerisierungsgrades ohne vorherige Desaktivierung gemeinsam mit dem nicht umgesetzten Ausgangsdiisocyanat destillativ vom Reaktionsprodukt abgetrennt.

Das erfindungsgemäße Verfahren wird vorzugsweise in Substanz durchgeführt. Es kann aber auch in Gegenwart von gegenüber Isocyanatgruppen inerten Lösungsmitteln durchgeführt werden. Geeignete Lösungsmittel sind beispielsweise Hexan, Toluol, Xylol, Chlorbenzol, Essigsäureethylester, Essigsäurebutylester, Ethylglykolacetat, Propylenglykolmonomethyletheracetat, Aceton, Methylisobutylketon, Methylenchlorid, N-Methylpyrrolidon oder beliebige Gemische derartiger Lösungsmittel.

Die Durchführung der Oligomerisierungsreaktion erfolgt beim erfindungsgemäßen Verfahren in an sich bekannter Weise, wie dies beispielsweise in den DE-A 16 70 720, 19 54 093, 34 37 635 oder 37 39 549 beschrieben ist.

Im allgemeinen wird so vorgegangen, daß man das Ausgangsdiisocyanat bzw. eine Mischung verschiedener Ausgangsdiisocyanate, gegebenenfalls unter Inertgas wie beispielsweise Stickstoff und gegebenenfalls in Gegenwart eines geeigneten Lösungsmittels der genannten Art auf eine Temperatur zwischen 20 und 100°C, vorzugsweise 40 bis 70°C erwärmt. Dann kann der gegebenenfalls mitzuverwendende, bevorzugt alkoholische Cokatalysator zugemischt werden. Im Anschluß hieran wird, gegebenenfalls nach Beendigung der spontan ablaufenden Reaktion zwischen Cokatalysator und Ausgangsdiisocyanat, ein Dimerisierungskatalysator der beispielhaft genannten Art in der oben angegebenen Menge zugegeben und die Reaktionstemperatur durch eine geeignete Maßnahme (Heizen oder Kühlen) auf eine Temperatur von 40 bis 120°C, vorzugsweise von 50 bis 80°C eingestellt. Im allgemeinen wird die Umsetzung bei Erreichen eines Oligomerisierungsgrades von 10 bis 60 %, vorzugsweise 10 bis 40 % beendet.

Unter "Oligomerisierungsgrad" ist der Prozentsatz der in der Ausgangsmischung vorliegenden Isocyanatgruppen zu verstehen, der während der erfindungsgemäßen Umsetzung (insbesondere durch Dimerisierung, daneben unter Trimerisierung und im Falle der Mitverwendung der beschriebenen, beispielsweise alkoholischen Cokatalysatoren durch Reaktion mit Isocyanatgruppen, beispielsweise unter Urethanisierung) verbraucht wird. Der genannte Oligomerisierungsgrad wird im allgemeinen nach einer Reaktionszeit von 1 bis 48, vorzugsweise 2 bis 24 Stunden erreicht. Die Beendigung der Umsetzung kann durch Zugabe eines Katalysatorgiftes der beispielhaft genannten Art und gegebenfalls nachfolgendes kurzzeitiges Erhitzen der Reaktionsmischung auf oberhalb 80°C, vorzugsweise oberhalb 120°C liegende Temperaturen erfolgen.

Das Reaktionsgemisch wird anschließend im allgemeinen durch Destillation im Hochvakuum, bevorzugt im Dünnschichtverdampfer, unter möglichst schonenden Bedingungen, beispielsweise bei einer Temperatur von 100 bis 160°C, vorzugsweise von 120 bis 140°C, von flüchtigen Bestandteilen (überschüssigen monomeren Diisocyanaten, gegebenenfalls mitverwendeten Lösungsmitteln und, bei Verzicht auf den Einsatz eines Katalysatorgiftes, gegebenenfalls aktivem Katalysator) befreit.

In einer anderen, wenn auch weniger bevorzugten Ausführungsform des erfindungsgemäßen Verfahrens werden die genannten flüchtigen Bestandteile durch Extraktion mit geeigneten gegenüber Isocyanatgruppen inerten Lösungsmitteln, beispielsweise aliphatischen oder cycloaliphatischen Kohlenwasserstoffen wie Pentan, Hexan, Heptan, Cyclopentan oder Cyclohexan vom Oligomerisierungsprodukt abgetrennt.

Auf diese Weise erhält man praktisch farblose, bei Raumtemperatur flüssige Uretdion- und gegebenfalls Isocyanuratgruppen aufweisende Polyisocyanatgemische, die im allgemeinen weniger als 5 Gew.-%, vorzugsweise weniger als 2 Gew.-%, besonders bevorzugt weniger als 1 Gew.-% an monomeren Ausgangsdiisocyanaten enthalten.

Diese monomerenarmen Polyisocyanatgemische werden nun zur Isolierung der reinen Bis(isocyanatoalkyl)-uretdione einem weiteren Destillationsschritt unterworfen. Als Destillationsapparaturen dienen dabei die üblichen Dünnschichtverdampfer, insbesondere Kurzwegverdampfer, wie sie beispielsweise auch für die oben beschriebene Abtrennung der monomeren Ausgangsdiisocyanate eingesetzt werden. Die Destillation wird im allgemeinen bei einer Temperatur oberhalb von 160°C, vorzugsweise im Bereich von 165 bis 220°C, besonders bevorzugt im Bereich von 170 bis 200°C im Hochvakuum, beispielsweise bei einem Druck von unter 1,0 mbar, vorzugsweise unter 0,5 mbar, besonders bevorzugt unter 0,2 mbar durchgeführt.

Die anfallenden Destillate bestehen zum überwiegenden Teil aus den gewünschten reinen Bis(isocyanatoalkyl)-monouretdionen neben geringeren Mengen an monomeren Ausgangsdiisocyanaten. Diese Monomeren, die zum einen aus den als Rohware eingesetzten oligomeren Polyisocyanatgemischen stammen, sich zum anderen aber auch während der Destillation durch thermische Uretdionspaltung in geringem Umfang zurückbilden, können, falls erforderlich, durch nochmalige Dünnschichtdestillation unter schonenden Bedingungen oder Extraktion entfernt werden, wie es bereits oben bei der sich an die Oligomerisierung anschließenden Abtrennung flüchtiger Bestandteile von der Reaktionsmischung beschrieben wurde.

Besonders vorteilhaft lassen sich die verschiedenen Destillationsschritte des erfindungsgemäßen Verfahrens auch in einer speziellen, aus drei hintereinandergeschalteten Verdampferstufen bestehenden Destillationsapparatur durchführen. Dabei wird in der ersten Stufe das durch Oligomerisierung der Diisocyanate erhaltene Reaktionsgemisch unter den oben genannten Bedingungen, bei möglichst niedrigen Temperaturen weitestgehend von flüchtigen Bestandteilen, insbesondere den monomeren Ausgangsdiisocyanaten, befreit und anschließend das monomerenarm als Destillationsrückstand ablaufende oligomere Polyisocyanatgemisch ohne weitere Isolierung in eine sich unmittelbar anschließende zweite Verdampfereinheit, vorzugsweise einen Kurzwegverdampfer, eingetragen. Hier erfolgt bei einer Temperatur oberhalb von 160°C dann die eigentliche erfindungswesentliche Abtrennung der reinen Monouretdiondiisocyanate von der Oligomerenmischung. Die Monouretdiondiisocyanate, die in dieser zweiten Destillationsstufe als Destillat anfallen, durchlaufen schließlich noch einen weiteren Destillationsschritt, in dem unter wiederum möglichst schonenden Bedingungen gegebenenfalls noch enthaltene Restmonomere entfernt werden.

Unabhängig von der Art der gewählten Ausführungsform erhält man erfindungsgemäß reine Bis(isocyanatoalkyl)-monouretdione in Form klarer, farbloser Flüssigkeiten, die nach gelpermeationschromatographischen Untersuchungen (GPC) zu über 97 Gew.-%, bevorzugt 98 bis 99,9 Gew.-% aus Dimeren der Ausgangsdiisocyanate bestehen, weniger als 2 Gew.-%, vorzugsweise weniger als 1,5 Gew.-%, besonders bevorzugt weniger als 1,0 Gew.-% an höhermolekularen Oligomeren und weniger als 0,5 Gew.-%, vorzugsweise weniger als 0,2 Gew.-%, besonders bevorzugt weniger als 0,1 Gew.-% an Restmonomeren enthalten und einen titrimetrisch bestimmten Gehalt an freien Isocyanatgruppen von 20,0 bis 30,1 Gew.-%, vorzugsweise von 24,9 bis 30,1 Gew.-%, besonders bevorzugt von 24,9 bis 25,1 Gew.-% aufweisen.

Diese reinen Monouretdiondiisocyanate zeichnen sich gegenüber den bekannten oligomeren Uretdionpolyisocyanaten des Standes der Technik insbesondere durch ihre sehr niedrigen Viskositäten von im allgemeinen weniger als 250 mPas (bei 23°C nach DIN 53 018) aus. Das im Falle des besonders bevorzugt eingesetzten Ausgangsdiisocyanates HDI nach dem erfindungsgemäßen Verfahren erhältliche Bis(6-isocyanatohexyl)-monouretdion weist beispielsweise eine Viskosität von lediglich 30 mPas (bei 23°C) auf.

Die nach der erfindungsgemäßen destillativen Abtrennung der Bis(isocyanatoalkyl)-monouretdione als Destillationsrückstand verbleibenden, an reinem Dimer abgereicherten uretdion- und gegebenenfalls isocyanuratgruppenhaltigen Oligomerengemische stellen praktisch farblose Harze dar, deren Viskositäten nur geringfügig über denen der aus den entsprechenden Ausgangsdiisocyanaten nach den bekannten Dimerisierungsverfahren des Standes der Technik erhältlichen Polyisocyanatgemische liegen. Sie enthalten im allgemeinen weniger als 0,5 Gew.-%, vorzugsweise weniger als 0,3 Gew.-%, besonders bevorzugt weniger als 0,2 Gew.-% an monomeren Ausgangsdiisocyanaten und weisen Gehalte an freien Isocyanatgruppen von 14,5 bis 24,5 Gew.-%, vorzugsweise von 19,0 bis 24,5 Gew.-%, besonders bevorzugt von 19,0 bis 20,5 Gew.-% auf.

Sowohl die nach dem erfindungsgemäßen Verfahren erhältlichen Bis(isocyanatoalkyl)-monouretdione als auch die als Koppelprodukt anfallenden "dimerarmen" Uretdion- und gegebenenfalls Isocyanuratgruppen aufweisenden Polyisocyanatgemische stellen wertvolle Ausgangsmaterialien zur Herstellung von Polyurethankunststoffen nach dem Polyadditionsverfahren, insbesondere zur Herstellung von Ein- oder Zweikomponenten-Polyurethanlacken, sowie in mit an sich bekannten Blockierungsmitteln für Isocyanatgruppen blockierter Form auch wertvolle Ausgangsmaterialien für Zweikomponenten-Polyurethan-Einbrennlacke dar.

Aufgrund ihrer außerordentlich niedrigen Viskosität sind die reinen Bis(isocyanatoalkyl)-uretdione insbesondere als Vernetzerkomponenten für lösemittelarme Zweikomponenten-Polyurethanlacke von großem Interesse. Ein weiteres bevorzugtes Einsatzgebiet der strikt linearen, difunktionellen Bis(isocyanatoalkyl)-monouretdione ist darüberhinaus die Herstellung uretdiongruppenhaltiger Polyaddtionsverbindungen als abspalterfreie Vernetzer für Hitze-vernetzbare Polyurethanpulverlacke (z. B. EP-A 45 994, EP-A 45 996, EP-A 45998 oder EP-A 639 598).

### Beispiele

Alle Prozentangaben beziehen sich auf das Gewicht, soweit nicht anders vermerkt.

### Beispiel 1

In 10 kg (59,5 mol) Hexamethylendiisocyanat (HDI) rührt man bei Raumtemperatur nacheinander 100 g (1,1 mol) 1,3-Butandiol und 30 g (0,15 mol) Tri-n-butylphosphin ein und erwärmt die Mischung anschließend auf 60°C. Nach einer Reaktionszeit von 4,5 Stunden beträgt der NCO-Gehalt des Reaktionsgemisches 42,5 %, entsprechend einem Oligomerisierungsgrad von 15 %. Die Reaktion wird durch Zugabe von 28 g (0,15 mol) Toluolsulfonsäuremethylester und einstündiges Erhitzen auf 80°C abgestoppt. Anschließend wird nicht umgesetztes überschüssiges HDI mit Hilfe eines Dünnschichtverdampfers bei einer Temperatur von 130°C und einem Druck von 0,5 mbar abdestilliert.

Das resultierende monomerenarme Oligomerengemisch weist einen Gehalt an freien NCO-Gruppen von 21,6 %, eine Viskosität (nach DIN 53 018) von 240 mPas (23°C), eine HAZEN-Farbzahl von ca. 50 auf und besitzt nach gelpermeationschromatographischen Untersuchungen (GPC) folgende Zusammensetzung:

| | |
|---|---|
| monomeres HDI: | 0,3 % |
| HDI-Uretdion (n=2): | 39,0 % |
| HDI-Isocyanurat (n=3): | 22,6 % |
| höhere Oligomere: | 38,1 % |

1000 g dieses Oligomerengemisches werden zur Isolierung des reinen Monouretdiondiisocyanates in einem handelsüblichen Kurzwegverdampfer bei einer Temperatur von 180°C und einem Druck von 0,1 mbar destilliert.

Als Harzablauf erhält man 670 g eines flüssigen Polyisocyanates mit einem Gehalt an freien NCO-Gruppen von 19,5 %, einer Viskosität von 690 mPas (23°C) und einer HAZEN-Farbzahl von 65, das sich laut GPC folgendermaßen zusammensetzt:

| | |
|---|---|
| monomeres HDI: | nicht nachweisbar |
| HDI-Uretdion (n=2): | 19,4 % |
| HDI-Isocyanurat (n=3): | 28,7 % |
| höhere Oligomere: | 51,9 % |

Das anfallende farblose Destillat wird zur Entfernung von monomerem HDI einer weiteren Dünnschichtdestillation bei einer Temperatur von 130°C und einem Druck von 0,2 mbar unterworfen.

Als Harzablauf dieser dritten Destillationsstufe erhält man schließlich 275 g des praktisch reinen Bis(6-isocyanatohexyl)-uretdions, entsprechend einer Ausbeute von 27,5 %, bezogen auf das eingesetzte monomerenarme Oligomerengemisch, mit einem NCO-Gehalt von 25,0 %, einer Viskosität von 30 mPas (23°C) und einer HAZEN-Farbzahl von ca. 10. Die folgende Tabelle zeigt die gelpermeationschromatographisch ermittelte Zusammensetzung des erfindungsgemäß erhaltenen Verfahrensproduktes:

| | |
|---|---|
| monomeres HDI: | nicht nachweisbar |
| HDI-Uretdion (n=2): | 99,1 % |
| HDI-Isocyanurat (n=3): | 0,3 % |
| höhere Oligomere: | 0,6 % |

### Beispiel 2

1000 g eines gemäß Beispiel 1 durch Oligomerisierung von HDI hergestellten monomerenarmen Oligomerengemisches wird in einem handelsüblichen Kurzwegverdampfer bei einer Temperatur von 190°C und einem Druck von 0,1 mbar destilliert.

Als Harzablauf erhält man 710 g eines flüssigen Polyisocyanates mit einem Gehalt an freien NCO-Gruppen von 19,9 %, einer Viskosität von 660 mPas (23°C) und einer HAZEN-Farbzahl von 70, das sich laut GPC folgendermaßen zusammensetzt:

| | |
|---|---|
| monomeres HDI: | 0,3 % |
| HDI-Uretdion (n=2): | 20,8 % |
| HDI-Isocyanurat (n=3): | 27,6 % |
| höhere Oligomere: | 51,3 % |

Das anfallende farblose Destillat wird zur Entfernung von monomerem HDI einer weiteren Dünnschichtdestillation bei einer Temperatur von 130°C und einem Druck von 0,1 mbar unterworfen.

Als Harzablauf dieser Destillationsstufe erhält man 223 g praktisch reines Bis(6-isocyanatohexyl)-uretdion, entsprechend einer Ausbeutevon 22,3 %, bezogen auf das eingesetzte monomerenarme Oligomerengemisch, mit einem NCO-Gehalt von 25,0 %, einer Viskosität von 32 mPas (23°C) und einer HAZEN-Farbzahl von 10. Die folgende Tabelle zeigt die gelpermeationschromatographisch ermittelte Zusammensetzung des erfindungsgemäß erhaltenen Uretdiondiisocyanates:

| | |
|---|---|
| monomeres HDI: | nicht nachweisbar |
| HDI-Uretdion (n=2): | 98,5 % |
| HDI-Isocyanurat (n=3): | 0,5 % |
| höhere Oligomere: | 1,0 % |

## Patentansprüche

1. Verfahren zur Herstellung von Bis(isocyanatoalkyl)-monouretdionen der Formel (I) 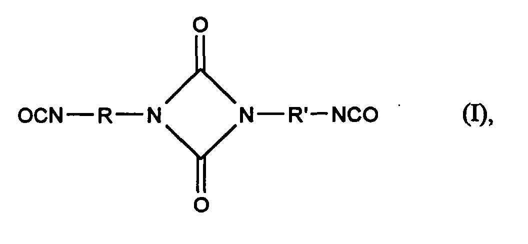 in welcher
R und R' für gleiche oder verschiedene, lineare oder verzweigte Alkylreste mit 4 bis 9 Kohlenstoffatomen stehen,
durch Oligomerisierung eines Teils der Isocyanatgruppen von Diisocyanaten oder Mischungen von Diisocyanaten der Formel (II)
OCN―R―NCO (II),
in welcher
R die bei Formel (I) genannte Bedeutung hat,
in Gegenwart von die Dimerisierung von Isocyanatgruppen beschleunigenden Katalysatoren oder Katalysatorsystemen, Abbruch der Oligomerisierungsreaktion bei einem gewünschten Oligomerisierungsgrad und Entfernung des nicht umgesetzten Diisocyanatüberschusses durch Extraktion oder Dünnschichtdestillation, und anschließende Abtrennung der Bis(isocyanatoalkyl)-monouretdione durch Dünnschicht-Destillation bei 160 bis 220°C und 0,01 bis 1,0 mbar.

2. Verfahren gemäß Anspruch 1, worin man lineare Diisocyanate mit 4 bis 6 Kohlenstoffatomen im Alkylteil einsetzt.

3. Verfahren gemäß Anspruch 1, worin man 1,6-Diisocyanatohexan einsetzt.

4. Verfahren gemäß Anspruch 1, worin man dreiwertigen Phosphor enthaltenden Katalysatoren verwendet.

5. Verfahren gemäß Anspruch 1,worin man Tributylphosphin oder Trioctylphosphin an Katalysatoren verwendet.

6. Bis(isocyanatoalkyl)-monouretdione der Formel I in Anspruch 1 mit einem Gehalt von mehr als 97 Gew.-%.

7. Verwendung der Bis(isocyanatoalkyl)-monouretdione gemäß Anspruch 1 als Ausgangskomponente bei der Herstellung von Polyurethankunststoffen.

8. Verwendung Bis(isocyanatoalkyl)-monouretdione gemäß Anspruch 1, gegebenenfalls in mit Blockierungsmitteln für Isocyanatgruppen blockierter Form, als Isocyanatkomponente in Polyurethanlacken.
